# EUROPEAN PATENT SPECIFICATION

(11) **EP 2 549 984 B1**
(45) Date of publication and mention of the grant of the patent: **18.03.2015**
(21) Application number: 11716189.3
(22) Date of filing: 23.03.2011
(51) Int. Cl.: A61K 9/24, A61K 31/485

(54) **DOUBLE-LAYER PHARMACEUTICAL FORMULATIONS CONTAINING OPIOID AGONISTS AND ANTAGONISTS**
DOPPELSCHICHTIGE PHARMAZEUTISCHE FORMULIERUNGEN MIT OPIOID-ANTAGONISTEN UND ANTAGONISTEN
PRÉPARATIONS PHARMACEUTIQUES DOUBLE-COUCHE CONTENANT DES AGONISTES ET DES ANTAGONISTES DES RÉCEPTEURS OPIOÏDES

(30) Priority: 24.03.2010 IT FI20100047
(43) Date of publication of application: 30.01.2013
(73) Proprietor: L. MOLTENI & C. DEI FRATELLI ALITTI - SOCIETA' DI ESERCIZIO S.P.A., 50018 Scandicci (IT)
(72) Inventor: ANGELI, Roberto, I-50018 Scandicci (IT); RAFFAELI, William, I-47923 Rimini (IT); RIGAMONTI, Maria Adele, I-50145 Firenze (IT)
(74) Representative: Brighenti, Livio
(86) International application number: PCT/EP2011/054463
(87) International publication number: WO 2011/117306

(56) References cited:
- WO-A1-96/02251
- WO-A1-03/013538
- WO-A1-2004/037260
- WO-A2-97/33566
- DE-A1- 4 325 465

## Description

### Field of the invention

The present invention relates to solid oral immediate-release tablets containing an opioid agonist and an opioid antagonist, and particularly to formulations in which said active ingredients are each contained in two separate layers.

### State of the art

It is common knowledge that opioid-based drugs are widely used to control painful syndromes, particularly when the pain cannot be controlled by less powerful therapies (as in the case of postoperative pain or chronic oncological and non-oncological pain).

On the other hand, the literature amply documents the numerous and even severe side-effects relating to the use of these drugs, e.g. drowsiness, nausea, vomiting, constipation, confusion, pruritus, headache, urinary retention, dysphoric reactions, respiratory depression and myoclonus. These side effects influence treatment with opioids, sometimes even prompting their suspension due to their poor tolerability, or negatively affecting the patient's quality of life, especially when long-term treatments are needed. As a result, given the importance of the use of opioids for pain control, intensive studies have obviously been conducted in an effort to overcome the above-mentioned drawbacks.

For instance, the use of an antiemetic such as metoclopramide has been considered to combat nausea and vomiting.

As an alternative, the administration of opioid antagonists simultaneously with the opioid agonists has been considered, e.g. in USP 5580876 or WO 96/02251). The Italian patent application MI2001A000907 reports on the use of very low doses of naltrexone in patients being treated with opioids to attenuate the unwanted side-effects.

DE 4325465 A1, WO 2004/037260 A1 and WO 03/013538 A1 disclose pharmaceutical compositions comprising a double-layer tablet wherein one layer includes an opioid agonist and a second layer comprises an opioid antagonist.

WO 97/33566 A2 relates to a pharmaceutical composition comprising a bilayered tablet wherein one layer includes an opioid agonist such as hydrocodone and a second layer comprises an opioid antagonist and, wherein the active ingredients are kept separate from each other.

The patent application EP 1 935 421 describes a controlled-release formulation containing an opioid agonist and an opioid antagonist, mixed together, and combined with compounds that modify the release of the two drugs; the quantity of antagonist in said formulations ranges from 100 to 1000 times less than that of the agonist.

Finally, WO 2005/107726 describes a composition containing opioid agonists and antagonists mixed together for the treatment of backache of arthritic origin.

In the light of the above-described state of the art, it is evident that the problem of pharmacologically controlling the side-effects of opioids, obviously while maintaining their analgesic efficacy, has yet to be fully overcome and different formulations are therefore needed, capable of dealing with the drawbacks that still exist in the currently known formulations, in which an opioid agonist and an opioid antagonist are administered to the patient simultaneously.

### Summary of the invention

Solid oral immediate-release formulations are described that are in the form of tablets containing an opioid agonist and an opioid antagonist, wherein said two active ingredients are maintained in separate layers.

### Detailed description of the invention

The present invention enables patients to be given formulations containing an agonist opioid and an antagonist capable of minimising the side effects relating to the administration of opioids. The object of the present invention is therefore pharmaceutical formulations for oral administration in tablet form comprising as active ingredients both an agonist opioid and an antagonist, wherein said active ingredients are contained in two separate layers.

The fact that the two active ingredients are in two separate layers within the same pharmaceutical formulation surprisingly proved capable of solving the problem of the side effects due to the use of opioids.

The simultaneous administration of the two active ingredients (with the antagonist in a minimal dosage) surprisingly results in a faster absorption of the antagonist, which goes to block the excitatory receptors responsible for the onset of the side-effects of the opioid agonist before it can bind to the inhibitory receptors and thereby exert its pain killing effect.

According to the invention, the term opioid agonists is used to mean a set of substances exhibiting the properties of opium, or morphine-like properties. Opiates are the opioid substances found in opium and their semi-synthetic derivatives. Possible examples of opioid agonists according to the invention include: oxycodone, hydromorphone, morphine, codeine, buprenorphine, fentanyl, methadone.

The term opioid antagonists is used to mean substances that occupy the opioid receptors without activating them and that are capable of weakening the response of the agonist opioid receptors.

Possible examples of opioid antagonists according to the invention include: naltrexone, naloxone.

According to a preferred embodiment of the invention, the quantity of opioid antagonist included in a formulation is in the range of 500 to 4000 times less than the quantity of opioid agonist.

In particular, according to a preferred embodiment of the invention, a formulation contains 0.005 mg of opioid antagonist and a quantity of agonist in the range of 2.5 - 20.0 mg.

Both the layers containing the two active ingredients comprise a mixture containing the common excipients used in the pharmacological sector, such as diluents (e.g. lactose), anticaking agents (e.g. cornstarch, croscarmellose sodium), release modifiers (e.g. Cutina® HR, Macrogol 6000), glidants (e.g. colloidal silica), lubricants (e.g. magnesium stearate), and any colouring agents allowable in pharmaceutical applications may also be added.

The advantageous characteristics of the formulations according to the invention have been further improved by the choice of specific excipients from among the many options available routinely used in solid oral formulations.

For the preparation of the layer containing the opioid agonist the following are preferred: SD lactose, pregelatinised corn starch, pigment, Macrogol 6000, Cutina® HR, and possibly also colloidal silica and magnesium stearate.

More preferably, the above-mentioned components are contained in the following percentages by weight, calculated on the total weight of the components of the layer concerned: SD lactose 40-60%, pregelatinised corn starch 10-20%, pigment 0.5-2%, Macrogol 6000 10-20%, Cutina® HR 5-20%, colloidal silica 0-2%, magnesium stearate 0-2%. The following are preferred for the preparation of the layer containing the opioid antagonist: Granulac 200 lactose, corn starch, croscarmellose sodium, polyvinylpyrrolidone K30, and possibly also anhydrous colloidal silica and magnesium stearate. More preferably, the above-mentioned components are contained in the following percentages by weight, calculated on the total weight of the components of the layer concerned: Granulac 200 lactose 30-80%, corn starch 5-10%, croscarmellose sodium 5-10%, polyvinylpyrrolidone K30 2-5%, anhydrous colloidal silica 0-2%, magnesium stearate 0-2%.

The two types of granules consisting of the above-mentioned components are compressed with the aid of a tablet press suitable for the preparation of double-layer tablets.

Then the double-layer tablets undergo film coating using a coating agent (e.g. HPMC Methocel E5) and a plasticiser (e.g. triethyl citrate). The double-layer tablets of the invention can be prepared, for instance, as outlined below.

### Layer containing the opioid agonist

The components of the mixture - agonist, diluent, anticaking agent, pigment (if any), release modifiers and glidant (if any) - are sieved and then mixed in a homogenizer, possibly adding the lubricant to the mixture while continuing to mix.

### Layer containing the opioid antagonist

The binder solution is prepared by dissolving the antagonist and the binder in water or alcohol. Then the product is granulated, proceeding as follows: the diluent and anticaking agents are mixed in a homogenizer, adding the binder solution.

The mixture is calibrated to the required dimensions and dried in the oven, then the dried granules are calibrated to the dimensions required, along with any anticaking agent and glidant. Then the final mixing process is completed, possibly adding the lubricant.

The double-layer tablets are prepared using a suitable double-layer tablet press to compress the two above-described compositions, one containing the opioid agonist and one containing the antagonist.

The double-layer tablets thus prepared may also be coated with suitable coating agents.

Below are several non-limiting examples to illustrate the present invention.

### Example 1

| Layer containing the opioid agonist | |
|---|---|
| Components | Quantity (mg) |
| oxycodone HCl | 2.5 |
| SD lactose | 105.0 |
| pregelatinised corn starch | 32.0 |
| Blend PB 24837 pink pigment | 1.50 |
| Macrogol 6000 | 28.0 |
| Cutina® HR | 21.75 |
| colloidal silica | 1.0 |
| magnesium stearate | 0.75 |
| total layer | 192.5 |

| Layer containing the opioid antagonist | |
|---|---|
| Components | Quantity (mg) |
| naltrexone HCl | 0.005 |
| Granulac 200 lactose | 64.0 |
| corn starch | 7.0 |
| croscarmellose sodium | 5.49 |
| polyvinylpyrrolidone K30 | 2.5 |
| colloidal silica anhydrous | 0.5 |
| magnesium stearate | 0.5 |
| total layer | 80.0 |
| coating | |
| HPMC Methocel E5 | 4.5 |
| triethyl citrate | 0.5 |
| total coating | 5.0 |
| Preparation | |

### (a) Layer containing the opioid agonist

The components (oxycodone HCl, SD lactose, pregelatinised corn starch, pigment, Macrogol 6000, Cutina® HR, colloidal silica) are sieved with a 20 mesh sieve, mixed for 120 rotations, then magnesium stearate is added and mixing continues for 25 revolutions.

### (b) Layer containing the opioid antagonist

The binder solution is prepared by dissolving naltrexone hydrochloride and polyvinylpyrrolidone K30 in water or alcohol. The Granulac 200 lactose, corn starch and croscarmellose sodium are mixed for 50 revolutions, then granulation proceeds with the previously-prepared binder solution.

The mixture is calibrated with a 5 mesh sieve and then dried in the oven (fluidized bed) at a temperature of 40°C, until a weight loss <1.5% has been achieved.

The dry granules are calibrated, together with the colloidal silica and croscarmellose sodium, through an 18 mesh sieve.

Then final mixing is done for 120 revolutions, before adding magnesium stearate and mixing again for 25 revolutions.

Preparation of the double-layer tablets

The two types of granules are compressed with the aid of a tablet press suitable for manufacturing double-layer tablets, the part containing the agonist weighing 192.5 mg and the part containing the antagonist weighing 80 mg.

Preparation of the film coating solution

In a suitable dissolver, transfer demineralised water, add HPMC Methocel E5 and mix for 45 minutes. Then add triethyl citrate and continue mixing.

### Film coating

The tablets are coated in the coating pan by spraying with the previously-prepared solution.

Operating in much the same way as described in example 1, formulations were obtained as described below.

### Example 2

| Components | Quantity (mg) |
|---|---|
| Layer containing the opioid agonist | |
| oxycodone HCl | 5.0 |
| SD lactose | 05.0 |
| pregelatinised corn starch, | 32.0 |
| Blend PB 24837 pink pigment | 1.50 |
| Macrogol 6000 | 28.0 |
| Cutina® HR | 21.75 |
| colloidal silica | 1.0 |
| magnesium stearate | 0.75 |
| total layer | 195.0 |

| Layer containing the opioid antagonist | |
|---|---|
| Components | Quantity (mg) |
| naltrexone HCl | 0.005 |
| Granulac 200 lactose | 64.0 |
| corn starch | 7.0 |
| croscarmellose sodium | 5.49 |
| polyvinylpyrrolidone K30 | 2.5 |
| anhydrous colloidal silica | 0.5 |
| magnesium stearate | 0.5 |
| total layer | 80.0 |
| coating | |
| HPMC Methocel E5 | 4.5 |
| triethyl citrate | 0.5 |
| total coating | 5.0 |

### Example 3

| Layer containing the opioid | |
|---|---|
| Components | Quantity (mg) |
| oxycodone HCl | 10.0 |
| SD lactose | 105.0 |
| pregelatinised corn starch | 32.0 |
| Blend PB 24837 pink pigment | 1.50 |
| Macrogol 6000 | 28.0 |
| Cutina® HR | 21.75 |
| colloidal silica | 1.0 |
| magnesium stearate | 0.75 |
| total layer | 200.0 |

| Layer containing the opioid antagonist | |
|---|---|
| Components | Quantity (mg) |
| naltrexone HCl | 0.005 |
| Granulac 200 lactose | 64.0 |
| corn starch | 7.0 |
| croscarmellose sodium | 5.49 |
| polyvinylpyrrolidone K30 | 2.5 |
| anhydrous colloidal silica | 0.5 |
| magnesium stearate | 0.5 |
| total layer | 80.0 |
| coating | |
| HPMC Methocel E5 | 4.5 |
| triethyl citrate | 0.5 |
| total coating | 5.0 |

### Example 4

| Layer containing the opioid | |
|---|---|
| Components | Quantity (mg) |
| oxycodone HCl | 20.0 |
| SD lactose | 105.0 |
| pregelatinised corn starch | 32.0 |
| Blend PB 24837 pink pigment | 1.50 |
| Macrogol 6000 | 28.0 |
| Cutina® HR | 21.75 |
| colloidal silica | 1.0 |
| magnesium stearate | 0.75 |
| total layer | 210.0 |

| Layer containing the opioid antagonist | |
|---|---|
| Components | Quantity (mg) |
| naltrexone HCl | 0.005 |
| Granulac 200 lactose | 64.0 |
| corn starch | 7.0 |
| croscarmellose sodium | 5.49 |
| polyvinylpyrrolidone K30 | 2.5 |
| anhydrous colloidal silica | 0.5 |
| magnesium stearate | 0.5 |
| total layer | |
| Coating | |
| HPMC Methocel E5 | 4.5 |
| triethyl citrate | 0.5 |
| total coating | 5.0 |

### Experimental assessment

Patients being treated with opioids underwent intrathecal screening, in which the evidence of side effects is extremely significant.

A dose of opioids was administered with or without naltrexone and the adverse events or side-effects and anti-nociceptive effects were monitored at several time points.

In particular, patients with chronic (non-oncological) spinal pain and oncological patients in the non-terminal phase with evidence of side effects after minimum doses of opioids were monitored. The results of the experimental assessment are summarised in the following tables.

| **Diagnosis** | **Side-effects of morphine** | **Side-effects of placebo** | **Side-effects of naltrexone** |
|---|---|---|---|
| Secondary malignancies of bone and marrow | pruritus (3) constipation | no effect | pruritus improved by 90% |
| Back pain | morphine | | naltrexone |
| | 30 min: vertigo (1) | no effect | h 17.30: tachycardia and chest pain |
| | 1 h: vertigo (1), nausea (1) | | |
| | | | h 18.30: tachycardia |
| | 2 h: vertigo (2), vomiting (2) | | |
| | 4 h: nausea (3), vertigo (2), vomiting (2) | | |
| | 8 h: pruritus (2) | | |
| | 24 h: nausea (2), vertigo (2) | | |
| Stenosis of the lumbar | headache, vomiting, nausea (4), sweating, pruritus (1) | no effect | naltrexone |
| spinal canal | | | nausea (1) |
| Nonspecific coccyx disorders | morphine 0 | no effect | morphine, bupivacaine, naltrexone |
| | 4h: recurrent vomiting | | |
| | 6h: vomiting (3), nausea (3) | | |
| | 8h: asthenia | | none |
| | Efficacy <30% | | Efficacy 30-50% |
| Lumbago | morphine | no effect | naltrexone |
| | 1 h: drowsiness (1) | | 1 h: drowsiness (1) |
| | | | 2 h: pruritus (2) |
| | 4 h: nausea (2), vomiting (2) | | 4 h: drowsiness (2), pruritus (2) |
| | 6 h: pruritus (3), urinary retention (3), nausea (3), vomiting (2) | | 6 h: pruritus (2), drowsiness (1) |
| | | | Efficacy |
| | | | 1 h: 30-50% |
| | 8 h: nausea (3), vomiting (2), pruritus (3), urinary retention (3) | | 4h-8h: >50% |
| Chronic pain after pelvis, sacral spine or coccyx trauma | morphine, bupivacaine | no effect | morphine, bupivacaine, naltrexone |
| | 1 h: confusion (2) | | 4h: pruritus (2), confusion (1) |
| | 2h: confusion(3), paresthesias | | 6h: pruritus (2), urinary retention (2) |
| | | | 8-24h: pruritus (1) |
| | 4h: confusion (2), pruritus (2) | | Efficacy: at rest |
| | 20h: urinary retention | | 1h: 20% |
| | | | 2h: 80% |
| | Efficacy: | | 4h: 100% |
| | 1-4h: 100% at rest | | 8-24h: 80% |
| | 6h: 100% under strain | | under strain |
| | | | 2h: 40% |
| | | | 8h: 80% |
| Lumboischialgia in diabetic patients (VAS 6-7) | nausea, constipation, loss of appetite, urinary retention, pruritus, drowsiness, moderate oedema | no effect | morphine hydrochloride bupivacaine HCl naltrexone |
| | | | 30 min after taking naltrexone: pruritus decreased by 100%, with onset of confusion (1), loss of appetite |
| Algoneurodystrophy | Morphine | no effect | morphine naltrexone |
| | 1 h: pruritus (3) | | |
| | 2h: pruritus (3) | | 6h: mild pruritus |
| | 6-8h: pruritus (3), urinary retention (1) | | Efficacy: 2h-8h 70% |
| | Efficacy: | | |
| | 2h: 30-40% | | |
| | 8h: 80% | | |
| | 14h: 70% | | |
| Lumbosacral spondylitis without myelopathy | morphine | no effect | morphine - naltrexone |
| | 2h-4h; nausea, vomiting | | nausea disappeared |
| | 6h-8h: vomiting | | |
| | Efficacy: | | Efficacy: |
| | 2h-24h: 80% | | 12h: 50-60% |
| Secondary bone and marrow malignancies | constipation (3), drowsiness | no effect | naltrexone |
| | (3), urinary retention (2) | | constipation (3), drowsiness (3), short-lived urinary retention |
| Cancer of the pancreas | | | morphine + naltrexone constipation |
| | nausea (3), constipation | no effect | |
| Persistent pain syndrome | vomiting (4), nausea, vertigo | no effect | naltrexone nausea, vertigo, vomiting, constipation, drowsiness, sweating |
| Secondary bone and marrow malignancies | morphine | no effect | morphine + naltrexone |
| | nausea (4), loss of appetite (4), vertigo (4), vomiting, constipation (1) | | loss of appetite (3), nausea (reduced by 50%), constipation (reduced by 60%), severe vertigo |
| | | | Efficacy: 100% |
| | Efficacy: 100% | | |
| Right lumboischialgia | | | naltrexone |
| | pruritus (4), nausea (2), vomiting (2), urinary retention (2) | no effect | |
| | | | pruritus (4), nausea (2), vomiting (2), urinary retention (2) |
| | | | pruritus (1), urinary retention (1) |
| Lumboischialgia | sweating (2), vertigo (2), pruritus (2), vomiting (3), nausea (2), | 2h: vertigo (1) | morphine bupivacaine HCl |
| | | 4h: vertigo (1) | naltrexone |
| | | | 2h: sweating (2), vertigo (2), pruritus (2) |
| | | | 4h: vomiting (3), nausea (2) |
| | | | 6h: vomiting (3), nausea (2) |
| | | | 8h: vomiting (4), nausea (2) |
| | | | Efficacy: |
| | | | 2h-4h: 80% |
| | | | 8h 100% |
| Neck pain | morphine + bupivacaine | no effect | morphine + naltrexone |
| | | | no side effects |
| | 1-4h: pruritus | | Efficacy: |
| | (2), 6-8h: pruritus (3). | | 1-2h 30% |
| | | | 4h 50% |
| | Efficacy: | | |
| | 1h-2h: 30% neck and 70% back | | |
| | 4h: 50% neck and 100% back | | |
| | 24h: 100% | | |
| Multiple sclerosis | nausea, loss of appetite, constipation, vertigo | no effect | morphine ropivacaine naltrexone nausea, loss of appetite, constipation, asthenia, drowsiness, vertigo |
| Diabetes mellitus type II | pruritus (4), nausea (3) | no effect | naltrexone |
| | | | benefit 100% |
| Dorsal spine pain | morphine + bupivacaine HCl | no effect | morphine + naltrexone |
| | pruritus, tingling, urinary retention | | pruritus and urinary retention improved |
| | | | Efficacy: |
| | Efficacy: | | 2h: 70% for spine, 50% for legs and feet, 100% at rest |
| | 4h: 50% for spine, 30% for legs and feet | | |
| | | | 4h: 50% for spine, 30% for legs |
| Spinal pain | morphine + bupivacaine | no effect | Naltrexone |
| | | | 4h: pruritus (1), numbness (1). |
| | 4h: pruritus (2), nausea (2) | | 6h: pruritus (1), numbness (1), nausea (1) |
| | 6h: pruritus (2), nausea (2), vomiting (1), urinary retention (2) | | 8h: nausea (1) |
| | | | Efficacy: |
| | | | 1 h-8h: at rest 100%, under strain 70% |
| | 8h: pruritus (2), nausea (2), urinary retention (2). | | |
| | 24h: pruritus (2) | | |
| | Efficacy: | | |
| | 1 h-8h: at rest 100%, under strain 70% | | |
| Persistent spinal pain syndrome of uncertain aetiology | morphine + bupivacaine | no effect | naltrexone |
| | | | none |
| | 1h: urinary retention (2) | | |
| | 2h: urinary retention (2) | | |
| | 4h: urinary retention (3) | | |

| | | | |
|---|---|---|---|
| *.0 = none; 1 = mild; 2 = moderate; 3 = intense; 4 = severe | | | |

## Claims

1. Immediate-release formulations in the form of double-layer tablets containing an opioid agonist and an opioid antagonist, wherein said active ingredients are kept separate from each other, each of them in one of said two layers and wherein the amount of opioid antagonist is 500-4000 times lower than that of the opioid agonist.

2. Formulations according to Claim 1 wherein said opioid agonists are chosen from among: oxycodone, hydromorphone, morphine, codeine, buprenorphine, methadone.

3. Formulations according to Claims 1 and 2, wherein said opioid antagonists are chosen from among: naltrexone and naloxone.

4. Formulations according to Claims 1 - 3, wherein the layer containing the agonist comprises: SD lactose, pregelatinised corn starch, pigment, Macrogol 6000, Cutina® HR, colloidal silica, magnesium stearate.

5. Formulations according to Claim 4, wherein said components of the layer containing the opioid agonist are present in the following percentages by weight, calculated on the total weight of the components of the aforesaid layer: SD lactose 40-60%, pregelatinised corn starch 10-20%, pigment 0.5-2%, Macrogol 6000 10-20%, Cutina® HR 5-20%, colloidal silica 0-2%, magnesium stearate 0-2%.

6. Formulations according to Claims 1 - 5, wherein the layer containing the opioid antagonist comprises: Granulac 200 lactose, corn starch, croscarmellose sodium, polyvinylpyrrolidone K30, colloidal silica, magnesium stearate.

7. Formulations according to Claim 6, wherein said components of the layer containing the opioid antagonist are present in the following percentages by weight, calculated on the total weight of the components of the aforesaid layer: Granulac 200 lactose 30-80%, corn starch 5-10%, croscarmellose sodium 5-10%, polyvinylpyrrolidone K30 2-5%, colloidal silica 0-2%, magnesium stearate 0-2%.

8. Formulations according to Claims 1 - 7, wherein the tablets are film-coated.

9. Formulations according to Claims 1 - 8 consisting of:
| (a) | |
|---|---|
| Layer containing the opioid agonist | |
| Components | Quantity (mg) |
| Oxycodone HCl | 2.5 |
| SD lactose | 105.0 |
| Pregelatinised corn starch | 32.0 |
| Blend PB 24837 pink pigment | 1.50 |
| Macrogol 6000 | 28.0 |
| Cutina® HR | 21.75 |
| Colloidal silica | 1.0 |
| Magnesium stearate | 0.75 |
| Total layer | 192.5 |
| Layer containing the opioid antagonist | |
|---|---|
| Components | Quantity (mg) |
| Naltrexone HCl | 0.005 |
| Granulac 200 lactose | 64.0 |
| Corn starch | 7.0 |
| Croscarmellose sodium | 5.49 |
| Polyvinylpyrrolidone K30 | 2.5 |
| Anhydrous colloidal silica | 0.5 |
| Magnesium stearate | 0.5 |
| Total layer | 80.0 |
| Coating | |
|---|---|
| HPMC Methocel E5 | 4.5 |
| Triethyl citrate | 0.5 |
| Total coating | 5.0 |
| | |
| (b) | |
|---|---|
| Layer containing the opioid agonist | |
| Components | Quantity (mg) |
| Oxycodone HCl | 5.0 |
| SD lactose | 105.0 |
| Pregelatinised corn starch | 32.0 |
| Blend PB 24837 pink pigment | 1.50 |
| Macrogol 6000 | 28.0 |
| Cutina® HR | 21.75 |
| Colloidal silica | 1.0 |
| Magnesium stearate | 0.75 |
| Total layer | 195.0 |
| Layer containing the opioid antagonist | |
|---|---|
| Components | Quantity (mg) |
| Naltrexone HCl | 0.005 |
| Granulac 200 lactose | 64.0 |
| Corn starch | 7.0 |
| Croscarmellose sodium | 5.49 |
| Polyvinylpyrrolidone K30 | 2.5 |
| Anhydrous colloidal silica | 0.5 |
| Magnesium stearate | 0.5 |
| Total layer | 80.0 |
| Coating | |
| HPMC Methocel E5 | 4.5 |
| Triethyl citrate | 0.5 |
| Total coating | 5.0 |
| | |
| (c) | |
|---|---|
| Layer containing the opioid agonist | |
| Components | Quantity (mg) |
| Oxycodone HCl | 10.0 |
| SD lactose | 105.0 |
| Pregelatinised corn starch | 32.0 |
| Blend PB 24837 pink pigment | 1.50 |
| Macrogol 6000 | 28.0 |
| Cutina® HR | 21.75 |
| Colloidal silica | 1.0 |
| Magnesium stearate | 0.75 |
| Total layer | 200.0 |
| Layer containing the opioid antagonist | |
| Components | Quantity (mg) |
|---|---|
| Naltrexone HCl | 0.005 |
| Granulac 200 lactose | 64.0 |
| Corn starch | 7.0 |
| Croscarmellose sodium | 5.49 |
| Polyvinylpyrrolidone K30 | 2.5 |
| Anhydrous colloidal silica | 0.5 |
| Magnesium stearate | 0.5 |
| Total layer | 80.0 |
| Coating | |
| HPMC Methocel E5 | 4.5 |
| Triethyl citrate | 0.5 |
| Total coating | 5.0 |
| | |
| (d) | |
|---|---|
| Layer containing the opioid agonist | |
| Components | Quantity (mg) |
| Oxycodone HCl | 20.0 |
| SD lactose | 105.0 |
| Pregelatinised corn starch | 32.0 |
| Blend PB 24837 pink pigment | 1.50 |
| Macrogol 6000 | 28.0 |
| Cutina® HR | 21.75 |
| Colloidal silica | 1.0 |
| Magnesium stearate | 0.75 |
| Total layer | 210.0 |
| Layer containing the opioid antagonist | |
|---|---|
| Components | Quantity (mg) |
| Naltrexone HCl | 0.005 |
| Granulac 200 lactose | 64.0 |
| Corn starch | 7.0 |
| Croscarmellose sodium | 5.49 |
| Polyvinylpyrrolidone K30 | 2.5 |
| Anhydrous colloidal silica | 0.5 |
| Magnesium stearate | 0.5 |
| Total layer | 80.0 |
| Coating | |
| HPMC Methocel E5 | 4.5 |
| Triethyl citrate | 0.5 |
| Total coating | 5.0 |

## Patentansprüche

1. Sofort freisetzende Formulierungen in der Form von Doppelschichttabletten, die einen Opioid-Agonisten und einen Opioid-Antagonisten enthalten, wobei die genannten aktiven Inhaltsstoffe voneinander getrennt gehalten werden, wobei jedes in einer der beiden Schichten enthalten ist, und, wobei die Menge an Opioid-Antagonist 500- bis 4000-mal niedriger ist als die des Opioid-Agonisten.

2. Formulierungen nach Anspruch 1, wobei die Opioid-Agonisten ausgewählt sind aus Oxycodon, Hydromorphon, Morphin, Codein, Buprenorphin, Methadon.

3. Formulierungen nach einem der Ansprüche 1 und 2, wobei die Opioid-Antagonisten ausgewählt sind aus Naltrexon und Naloxon.

4. Formulierungen nach einem der Ansprüche 1 bis 3, wobei die Schicht, welche den Agonisten enthält, SD Lactose, vorgelierte Maisstärke, Pigment, Macrogol 6000, Cutina® HR, kolloidales Silica, Magnesiumstearat umfasst.

5. Formulierungen nach Anspruch 4, wobei die Komponenten der Schicht, welche den Opioid-Agonisten enthält, in den folgenden Gew.-%, berechnet auf Basis des Gesamtgewichts der Bestandteile der zuvor genannten Schicht, vorliegen: SD Lactose 40-60%, vorgelierte Maisstärke 10-20%, Pigment 0.5-2%, Macrogol 6000 10-20%, Cutina® HR 5-20%, kolloidales Silica 0-2%, Magnesiumstearat 0-2%.

6. Formulierungen nach einem der Ansprüche 1 bis 5, wobei die den Opioid-Antagonist enthaltende Schicht umfasst: Granulac 200 Lactose, Maisstärke, Croscarmellose-Natrium, Polyvinylpyrrolidon K30, kolloidales Silica, Magnesiumstearat.

7. Formulierungen nach Anspruch 6, wobei die Komponenten der Schicht, welche den Opioid-Antagonisten enthält, in den folgenden Gew.-%, berechnet auf Basis des Gesamtgewichts der Bestandteile der zuvor genannten Schicht, vorliegen: Granulac 200 Lactose 30-80%, Maisstärke 5-10%, Croscarmellose-Natrium 5-10%, Polyvinylpyrrolidon K30 2-5%, kolloidales Silica 0-2%, Magnesiumstearat 0-2%.

8. Formulierungen nach einem der Ansprüche 1 bis 7, wobei die Tabletten filmbeschichtet sind.

9. Formulierungen nach einem der Ansprüche 1 bis 8, bestehend aus:
| (a) | |
|---|---|
| Opioid-Agonist enthaltende Schicht | |
| Bestandteile | Menge (mg) |
| Oxycodon HCl | 2,5 |
| SD Lactose | 105,0 |
| vorgelierte Maisstärke | 32,0 |
| Mischung PB 24837 Pink Pigment | 1,50 |
| Macrogol 6000 | 28,0 |
| Cutina® HR | 21,75 |
| kolloidales Silica | 1,0 |
| Magnesiumstearat | 0,75 |
| Gesamte Schicht | 192,5 |
| Opioid-Antagonist enthaltende Schicht | |
|---|---|
| Bestandteile | Menge (mg) |
| Naltrexon HCl | 0,005 |
| Granulac 200 Lactose | 64,0 |
| Maisstärke | 7,0 |
| Croscarmellose-Natrium | 5,49 |
| Polyvinylpyrrolidon K30 | 2,5 |
| Wasserfreies kolloidales Silica | 0,5 |
| Magnesiumstearat | 0,5 |
| Gesamte Schicht | 80,0 |
| Beschichtung | |
| HPMC Methocel E5 | 4,5 |
| Triethylcitrat | 0,5 |
| Gesamte Beschichtung | 5,0 |
| | |
| (b) | |
|---|---|
| Opioid-Agonist enthaltende Schicht | |
| Bestandteile | Menge (mg) |
| Oxycodon HCl | 5,0 |
| SD Lactose | 105,0 |
| vorgelierte Maisstärke | 32,0 |
| Mischung PB 24837 Pink Pigment | 1,50 |
| Macrogol 6000 | 28,0 |
| Cutina® HR | 21,75 |
| kolloidales Silica | 1,0 |
| Magnesiumstearat | 0,75 |
| Gesamte Schicht | 195,0 |
| Opioid-Antagonist enthaltende Schicht | |
| Bestandteile | Menge (mg) |
| Naltrexon HCl | 0,005 |
| Granulac 200 Lactose | 64,0 |
| Maisstärke | 7,0 |
| Croscarmellose-Natrium | 5,49 |
| Polyvinylpyrrolidon K30 | 2,5 |
| Wasserfreies kolloidales Silica | 0,5 |
| Magnesiumstearat | 0,5 |
| Gesamte Schicht | 80,0 |
| Beschichtung | |
| HPMC Methocel E5 | 4,5 |
| Triethylcitrat | 0,5 |
| Gesamte Beschichtung | 5,0 |
| | |
| (c) | |
|---|---|
| Opioid-Agonist enthaltende Schicht | |
| Bestandteile | Menge (mg) |
| Oxycodon HCl | 10,0 |
| SD Lactose | 105,0 |
| vorgelierte Maisstärke | 32,0 |
| Mischung PB 24837 Pink Pigment | 1,50 |
| Macrogol 6000 | 28,0 |
| Cutina® HR | 21,75 |
| kolloidales Silica | 1,0 |
| Magnesiumstearat | 0,75 |
| Gesamte Schicht | 200,0 |
| Opioid-Antagonist enthaltende Schicht | |
|---|---|
| Bestandteile | Menge (mg) |
| Naltrexon HCl | 0,005 |
| Granulac 200 Lactose | 64,0 |
| Maisstärke | 7,0 |
| Croscarmellose-Natrium | 5,49 |
| Polyvinylpyrrolidon K30 | 2,5 |
| Wasserfreies kolloidales Silica | 0,5 |
| Magnesiumstearat | 0,5 |
| Gesamte Schicht | 80,0 |
| Beschichtung | |
| HPMC Methocel E5 | 4,5 |
| Triethylcitrat | 0,5 |
| Gesamte Beschichtung | 5,0 |
| | |
| (d) | |
|---|---|
| Opioid-Agonist enthaltende Schicht | |
| Bestandteile | Menge (mg) |
| Oxycodon HCl | 20,0 |
| SD Lactose | 105,0 |
| vorgelierte Maisstärke | 32,0 |
| Mischung PB 24837 pink pigment | 1,50 |
| Macrogol 6000 | 28,0 |
| Cutina® HR | 21,75 |
| kolloidales Silica | 1,0 |
| Magnesiumstearat | 0,75 |
| Gesamte Schicht | 210,0 |
| Opioid-Antagonist enthaltende Schicht | |
|---|---|
| Bestandteile | Menge (mg) |
| Naltrexon HCl | 0,005 |
| Granulac 200 Lactose | 64,0 |
| Maisstärke | 7,0 |
| Croscarmellose-Natrium | 5,49 |
| Polyvinylpyrrolidon K30 | 2,5 |
| Wasserfreies kolloidales Silica | 0,5 |
| Magnesiumstearat | 0,5 |
| Gesamte Schicht | 80,0 |
| Beschichtung | |
| HPMC Methocel E5 | 4,5 |
| Triethylcitrat | 0,5 |
| Gesamte Beschichtung | 5,0 |

## Revendications

1. Formulations à libération immédiate sous forme de comprimés double couche contenant un agoniste des récepteurs opioïdes et un antagoniste des récepteurs opioïdes, où lesdits ingrédients actifs sont conservés séparément les uns des autres, chacun d'eux dans l'une desdites deux couches et où la quantité de l'antagoniste des récepteurs opioïdes est 500 à 4 000 fois inférieure à celle de l'agoniste des récepteurs opioïdes.

2. Formulations selon la revendication 1, où lesdits agonistes des récepteurs opioïdes sont choisis parmi : l'oxycodone, l'hydromorphone, la morphine, la codéine, la buprénorphine, la méthadone.

3. Formulations selon les revendications 1 et 2, où lesdits antagonistes des récepteurs opioïdes sont choisis parmi : la naltrexone et la naloxone.

4. Formulations selon les revendications 1 à 3, où la couche contenant l'agoniste comprend : du lactose SD, de l'amidon de maïs prégélatinisé, un pigment, du Macrogol 6000, du Cutina® HR, de la silice colloïdale, du stéarate de magnésium.

5. Formulations selon la revendication 4, où lesdits composants de la couche contenant l'agoniste des récepteurs opioïdes sont présents dans les pourcentages en poids suivants, calculés par rapport au poids total des composants de la couche susmentionnée : 40 à 60 % de lactose SD, 10 à 20 % d'amidon de maïs prégélatinisé, 0,5 à 2 % de pigment, 10 à 20 % de Macrogol 6000, 5 à 20 % de Cutina® HR, 0 à 2 % de silice colloïdale, 0 à 2 % de stéarate de magnésium.

6. Formulations selon les revendications 1 à 5, où la couche contenant l'antagoniste des récepteurs opioïdes comprend : du lactose Granulac 200, de l'amidon de maïs, de la croscarmellose sodique, de la polyvinylpyrrolidone K30, de la silice colloïdale, du stéarate de magnésium.

7. Formulations selon la revendication 6, où lesdits composants de la couche contenant l'antagoniste des récepteurs opioïdes sont présents dans les pourcentages en poids suivants, calculés par rapport au poids total des composants de la couche susmentionnée : 30 à 80 % de lactose Granulac 200, 5 à 10 % d'amidon de maïs, 5 à 10 % de croscarmellose sodique, 2 à 5 % de polyvinylpyrrolidone K30, 0 à 2 % de silice colloïdale, 0 à 2 % de stéarate de magnésium.

8. Formulations selon les revendications 1 à 7, où les comprimés sont enrobés d'un film.

9. Formulations selon les revendications 1 à 8 qui consistent en :
| (a) | |
|---|---|
| Couche contenant l'agoniste des récepteurs opioïdes | |
| Composants | Quantité (mg) |
| Oxycodone HCl | 2,5 |
| Lactose SD | 105,0 |
| Amidon de maïs prégélatinisé | 32,0 |
| Mélange de pigment rose PB 24837 | 1,50 |
| Macrogol 6000 | 28,0 |
| Cutina® HR | 21,75 |
| Silice colloïdale | 1,0 |
| Stéarate de magnésium | 0,75 |
| Total couche | 192,5 |
| Couche contenant l'antagoniste des récepteurs opioïdes | |
|---|---|
| Composants | Quantité (mg) |
| Naltrexone HCl | 0,005 |
| Lactose Granulac 200 | 64,0 |
| Amidon de maïs | 7,0 |
| Croscarmellose sodique | 5,49 |
| Polyvinylpyrrolidone K30 | 2,5 |
| Silice colloïdale anhydre | 0,5 |
| Stéarate de magnésium | 0,5 |
| Total couche | 80,0 |
| Enrobage | |
| HPMC Methocel E5 | 4,5 |
| Citrate de triéthyle | 0,5 |
| Total enrobage | 5,0 |
| (b) | |
|---|---|
| Couche contenant l'agoniste des récepteurs opioïdes | |
| Composants | Quantité (mg) |
| Oxycodone HCl | 5,0 |
| Lactose SD | 105,0 |
| Amidon de maïs prégélatinisé | 32,0 |
| Mélange de pigment rose PB 24837 | 1,50 |
| Macrogol 6000 | 28,0 |
| Cutina® HR | 21,75 |
| Silice colloïdale | 1,0 |
| Stéarate de magnésium | 0,75 |
| Total couche | 195,0 |
| Couche contenant l'antagoniste des récepteurs opioïdes | |
|---|---|
| Composants | Quantité (mg) |
| Naltrexone HCl | 0,005 |
| Lactose Granulac 200 | 64,0 |
| Amidon de maïs | 7,0 |
| Croscarmellose sodique | 5,49 |
| Polyvinylpyrrolidone K30 | 2,5 |
| Silice colloïdale anhydre | 0,5 |
| Stéarate de magnésium | 0,5 |
| Total couche | 80,0 |
| Enrobage | |
| HPMC Methocel E5 | 4,5 |
| Citrate de triéthyle | 0,5 |
| Total enrobage | 5,0 |
| (c) | |
|---|---|
| Couche contenant l'agoniste des récepteurs opioïdes | |
| Composants | Quantité (mg) |
| Oxycodone HCl | 10,0 |
| Lactose SD | 105,0 |
| Amidon de maïs prégélatinisé | 32,0 |
| Mélange de pigment rose PB 24837 | 1,50 |
| Macrogol 6000 | 28,0 |
| Cutina® HR | 21,75 |
| Silice colloïdale | 1,0 |
| Stéarate de magnésium | 0,75 |
| Total couche | 200,0 |
| Couche contenant l'antagoniste des récepteurs opioïdes | |
|---|---|
| Composants | Quantité (mg) |
| Naltrexone HCl | 0,005 |
| Lactose Granulac 200 | 64,0 |
| Amidon de maïs | 7,0 |
| Croscarmellose sodique | 5,49 |
| Polyvinylpyrrolidone K30 | 2,5 |
| Silice colloïdale anhydre | 0,5 |
| Stéarate de magnésium | 0,5 |
| Total couche | 80,0 |
| Enrobage | |
| HPMC Methocel E5 | 4,5 |
| Citrate de triéthyle | 0,5 |
| Total enrobage | 5,0 |
| (d) | |
|---|---|
| Couche contenant l'agoniste des récepteurs opioïdes | |
| Composants | Quantité (mg) |
| Oxycodone HCl | 20,0 |
| Lactose SD | 105,0 |
| Amidon de maïs prégélatinisé | 32,0 |
| Mélange de pigment rose PB 24837 | 1,50 |
| Macrogol 6000 | 28,0 |
| Cutina® HR | 21,75 |
| Silice colloïdale | 1,0 |
| Stéarate de magnésium | 0,75 |
| Total couche | 210,0 |
| Couche contenant l'antagoniste des récepteurs opioïdes | |
|---|---|
| Composants | Quantité (mg) |
| Naltrexone HCl | 0,005 |
| Lactose Granulac 200 | 64,0 |
| Amidon de maïs | 7,0 |
| Croscarmellose sodique | 5,49 |
| Polyvinylpyrrolidone K30 | 2,5 |
| Silice colloïdale anhydre | 0,5 |
| Stéarate de magnésium | 0,5 |
| Total couche | 80,0 |
| Enrobage | |
| HPMC Methocel E5 | 4,5 |
| Citrate de triéthyle | 0,5 |
| Total enrobage | 5,0 |
